# EUROPEAN PATENT APPLICATION

(11) **EP 4 454 639 A1**
(43) Date of publication of application: **30.10.2024**
(21) Application number: 24169892.7
(22) Date of filing: 12.04.2024
(51) Int. Cl.: A61K 9/08, A61K 47/32, A61K 47/36, A61K 31/185, A61P 27/04

(54) **OPHTHALMIC COMPOSITION COMPRISING CARBOMER AND TAURINE**

(30) Priority: 14.04.2023 EP 23167977
(71) Applicant: Omnivision GmbH, 82178 Puchheim (DE)
(72) Inventor: Melzer, Benedikt, 82178 Puchheim (DE); Hoffmann, Patrick, 82178 Puchheim (DE); Hoffmann, Burkhardt, 82178 Puchheim (DE); Kohl, Tobias, 82178 Puchheim (DE)
(74) Representative: Wächtershäuser & Hartz Patentanwaltspartnerschaft mbB

(57) **Abstract**

The invention provides an aqueous ophthalmic composition comprising (i) polyacrylic acid and (ii) taurine. The composition may be used in the treatment or prevention of dry eyes or of epithelial defects in the cornea.

## Description

### FIELD OF THE INVENTION

The present invention provides an aqueous ophthalmic composition comprising a polyacrylic acid (or a salt thereof), such as carbomer, and taurine, preferably further comprising panthenol. In another embodiment, the invention provides an aqueous ophthalmic composition comprising taurine and panthenol. The compositions are useful as pharmaceutical compositions and/or medical devices. The compositions may be used in the treatment or prevention of, inter alia, dry eyes (such as dry eye syndrome) or of epithelial defects in the cornea.

### BACKGROUND OF THE INVENTION

Several components of the ocular surface contribute to maintaining and protecting a smooth refractive layer to facilitate the optimal eyesight. At the air-water interface, the tear film lipid layer (TFLL), a mixture of lipids and proteins, plays a key role in tear surface tension and is important for the physiological hydration of the ocular surface and for ocular homeostasis. Alterations in tear fluid rheology, differences in lipid composition, or downregulation of specific tear proteins are found in most types of ocular surface disease, including dry eye disease (DED). Artificial tears have long been a first line treatment in DED and aim to replace or supplement natural tears.

Dry eye syndrome (also known as keratoconjunctivitis sicca), is a chronic and usually progressive disease caused by multiple etiologies and associated with a range of different symptoms. Recently, science is gaining more and more insight into the exact pathogenesis of the disease up to the developmental mechanisms on a molecular biological level. According to current knowledge, there are a number of mechanisms to which the development of the disease is attributed. These include a decrease or complete cessation of tear film secretion, instability of the resulting tear film, hyperosmolarity of the tear film, worsening of tear film break-up time (TBUT), onset of inflammatory processes, disruption of meibomian gland function etc.

Current treatment of dry eye syndrome is gradual, with the main focus on artificial tears, mostly in the form of eye drops. These generally contain hydrophilic polymers as gelling agents, such as carbomer (e.g. Visco-Vision^{®} Gel), hyaluronic acid (e.g. Hylo-Vision^{®}), and cellulose ethers in varying concentrations, that have film-forming properties on the cornea when instilled into an eye (on the eye surface).

Notwithstanding the undisputed efficacy of this approach, there remain problems. For example, the time artificial tears remain on the eye surface can be relatively short or they dry relatively fast, whereby the wetting effect does not last sufficiently long. As a result, artificial tears have to be (re-)administered regularly, which is not convenient for the patient.

CN 114 425 033 A describes an ophthalmic gel containing mesenchymal stem cell exosomes with sodium hyaluronate as a matrix. Examples describe gels containing carbomer and taurine and sodium chloride as an isotonicity regulator in a solvent that is physiological saline.

It is an object of the present invention to provide an ophthalmic composition usable as artificial tears, having a long-lasting effect such as longer remaining time on the cornea or less tendency to dry out after having been applied on the cornea, and a long-lasting wetting effect. The artificial tears should have a long or better adhesion to the surface of the eye, notably to the mucin layer on the outer surface of the cornea. Further, artificial tears should have a high resistance to drying, which allows reducing the frequency of administration for a given state of DED and/or enhancing the efficacy in the treatment or prevention of DED.

### SUMMARY OF THE INVENTION

In order to solve the above problems, the present invention provides:
(1) An aqueous ophthalmic composition comprising:
   (i) polyacrylic acid and
   (ii) taurine.
(2) The ophthalmic composition according to (1), further comprising
   (iii) panthenol.
(3) An aqueous ophthalmic composition comprising:
   (i) polyacrylic acid or a salt thereof and
   (ii) taurine,
   wherein the concentration of alkali metal chloride, notably of sodium and/or potassium chloride, in the composition is 0.4 % w/v or less, preferably 0.2 % w/v or less, more preferably 0.1% w/v or less, even more preferably 0.05% w/v or less, and even more preferably 0.02% w/v or less.
(4) An aqueous ophthalmic composition comprising:
   (i) polyacrylic acid or a salt thereof and
   (ii) taurine,
   wherein the composition comprises alkali metal chloride, notably sodium and/or potassium chloride, in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v.
(5) The ophthalmic composition according to any one of (1) to (4), further comprising
   (iv) hyaluronic acid or a salt thereof.
(6) The ophthalmic composition according to any one of (1) to (5), further comprising EDTA or a salt thereof.
(7) The ophthalmic composition according to any one of (1) to (6), further comprising a tonicity agent, such as sorbitol.
(8) The ophthalmic composition according to (7), wherein the concentration of sodium chloride is 0.4 % w/v or less, preferably 0.2% w/v or less.
(9) The ophthalmic composition according to any one of (1) to (8), wherein the composition is an aqueous solution having an osmolality of from 200 to 350 mOsm/kg.
(10) An aqueous ophthalmic composition according to any one of (1) to (8), wherein the composition is an aqueous solution having an osmolality of from 120 to 350 mOsm/kg, preferably 150 to 300 mOsm/kg, even more preferably from 170 to 280 mOsm/kg, even more preferably from 180 to 270 mOsm/kg, and most preferably from 190 to 260 mOsm/kg.
(11) The ophthalmic composition according to any one of (1) to (10), wherein the concentration of alkali metal ions, notably of sodium and/or potassium ions, in the composition is at most 70 mM, preferably at most 35 mM, more preferably at most 20 mM, even more preferably at most 10 mM, and most preferably at most 3 mM.
(12) The ophthalmic composition according to any one of claims (1) to (11), wherein the concentration of alkali metal ions, notably of sodium and/or potassium ions, in the composition is from 2 to 35 mM, preferably from 4 to 25 mM, more preferably from 5 to 25 mM, even more preferably from 5 to 17 mM, and most preferably from 5 to 14 mM.
(13) The ophthalmic composition according to any one of (1) to (12), having a viscosity of from 10 to 3000 mPa s, preferably of from 50 to 2000 mPa s, and more preferably from 100 to 1500 mPa·s at 20°C, determined using a rotational viscometer.
(14) The ophthalmic composition according to any one of (1) to (12), having at a shear rate of 100 s⁻¹ a viscosity of from 10 to 3000 mPa s, preferably of from 50 to 2000 mPa s, and more preferably from 100 to 1500 mPa·s at 25°C, determined using a rotational viscometer.
(15) The ophthalmic composition according to any one of (1) to (12), having at a shear rate of 100 s⁻¹ a viscosity of from 10 to 400 mPa s, preferably of from 20 to 300 mPa s, and more preferably from 30 to 200 mPa·s at 25°C, determined using a rotational viscometer.
(16) The ophthalmic composition according to any one of (1) to (15), having a pH of from 5.0 to 7.5, preferably from 5.5 to 7.2, more preferably from 6.0 to 7.0.
(17) The aqueous ophthalmic composition according to any one of (1) to (16), comprising:
   (i) 0.05 to 0.4 % w/v polyacrylic acid or a salt thereof and
   (ii) 0.2 to 3.0 % w/v taurine, preferably 0.3 to 2.5 % w/v taurine, more preferably from 0.4 to 2.2 % w/v taurine; in one embodiment, the ophthalmic composition comprises from 0.2 to 1.0 % w/v taurine.
(18) The aqueous ophthalmic composition according to (17), further comprising
   (iii) 0.5 to 4.0 % w/v panthenol.
(19) The aqueous ophthalmic composition according to (17) or (18), further comprising
   (iv) 0.05 to 0.3 % w/v hyaluronic acid or a salt thereof.
(20) The ophthalmic composition according to any one of (1) to (19), said polyacrylic acid having a mean weight average molecular weight of from 500,000 to 5,000,000 Da, preferably from 1,000,000 to 4,000,000 Da.
(21) An aqueous ophthalmic composition comprising
   (i) 0.1 to 0.3 % w/v polyacrylic acid or a salt thereof,
   (ii) 0.2 to 3.0 % w/v taurine, preferably 0.3 to 2.5 % w/v taurine, more preferably from 0.4 to 2.2 % w/v taurine; in one embodiment, from 0.2 to 1.0 % w/v taurine, and
   (iii) 0.5 to 3.0 % w/v panthenol, and
   wherein the concentration of alkali metal ions in the composition is preferably at most 70 mM, preferably at most 35 mM, more preferably at most 10 mM, and most preferably at most 4 mM.
(22) The aqueous ophthalmic composition according to (21), further comprising 0.05 to 0.3 % w/v hyaluronic acid or a salt thereof.
(23) The ophthalmic composition according to any one of (1) to (22), which is an aqueous solution of a single phase, preferably does not contain lipids (triacylglycerides) nor phospholipids (diacylphosphoglycerides).
(24) The ophthalmic composition according to any one of (1) to (23), further comprising an active pharmaceutical ingredient.
(25) The ophthalmic composition according to any one of (1) to (24) for use in the treatment or prevention of dry eyes or of epithelial defects in the cornea.
(26) The ophthalmic composition for the use according to (25), wherein said ophthalmic composition is applied to the eyes of a human subject by instillation.
(27) An aqueous composition comprising:
   (ii) 0.2 to 1.0 % w/v taurine and
   (iii) 0.5 to 3.0 % w/v panthenol
   preferably for the production of an ophthalmic composition.

The inventors have surprisingly found that strong decrease of the viscosity of carbomer solutions in water by the presence of sodium chloride or other alkali metal halides can be reduced or prevented, if the alkali metal halide such as NaCl is avoided or reduced and taurine is used as a tonicity agent. Even more surprisingly, at a high shear rate of 100 s⁻¹ (approaching that the shear rate of tear film occurring in the eyes upon blinking) and low salt concentrations (e.g. 0.025 to 0.1 %), the viscosity of aqueous carbomer solutions is even higher if taurine is quite highly concentrated in the ophthalmic composition, such as at or on the order to 2 % w/v. Thus, the strong decrease of the viscosity of carbomer solutions by the presence of salt can partly be reversed by the presence of taurine. Due to the higher viscosity, a longer remaining time on the surface of the eye can be achieved.

### BRIEF DESCRIPTION OF THE FIGURES

**Fig. 1** shows the viscosity of an aqueous carbomer solution in the absence of taurine (diamonds) and in the presence of 2% w/v taurine (crosses) dependent on the concentration against NaCl. NaCl concentrations are varied between 0 and 0.1 % w/w. Viscosity values are given in Pa s. The viscosity is measured at a shear rate of 100 s⁻¹ at 20 °C.

**Fig. 2** Same as in Fig. 1, but on different scale.

### DETAILED DESCRIPTION OF THE INVENTION

The aqueous ophthalmic composition of the invention contains at least polyacrylic acid (carbomer) and taurine. Taurine is 2-aminoethane-1-sulfonic acid. The skilled person understands that the protonation state and/or charge of taurine depends on the pH of the aqueous solution in which it is dissolved. Therefore, the term taurine comprises salts of taurine. However, where the concentration of taurine in the ophthalmic composition is given, the weight or weight percentage is in terms of the mass of 2-aminoethane-1-sulfonic acid. The concentration of taurine in the aqueous ophthalmic composition is generally from 0.1 to 2.0 % w/v, preferably from 0.2 to 1.0 % w/v taurine, more preferably from 0.3 to 0.7 % w/v of the aqueous ophthalmic composition.

The use of polyacrylic acid in ophthalmic compositions is generally known, e.g. from Amin PD et al., Studies on gel tears, Drug Dev Ind Pharm 1996, 22(7): 735-739; Ünlü N. et al. Formulation of carbopol 940 ophthalmic vehicles, and in vitro evaluation of the influence of simulated lacrimal fluid on their physico-chemical properties, Pharmazie 1991, 46: 784-788; and from Deshpande SG, Shirolkar S. Sustained release ophthalmic formulations of pilocarpine. J Pharm Pharmacol 1989; 41: 197-200.

The polyacrylic acid used in the aqueous ophthalmic composition is not particularly limited and is as frequently used in artificial tears. The polyacrylic acid may be present in the ophthalmic composition in a concentration of from 0.05 to 1.0 % w/v, preferable of from 0.15 to 0.9 % w/v, and more preferably of from 0.3 to 0.8 % w/v. Accordingly, the concentration is indicated with reference to the acid form of the polyacrylic acid (as opposed to salts thereof). Preferred polyacrylic acid is carbomer. The weight average molecular weight of the polyacrylic acid may be from 500,000 to 5,000,000 Da, preferably from 1,000,000 to 4,000,000 Da. The polyacrylic acid for use in the invention includes cross-linked polyacrylic acid, such as those known as carbomers. Carbomers may be cross-linked e.g. with allyl sucrose or allylpentaerythritol. A preferred carbomer for use in the compositions of the present invention is a polymer of acrylic acid cross-linked with allyl sucrose or allylpentaerythritol. Examples of carbomers suitable for use in the invention are carbomer 910, carbomer 940, carbomer 934P, carbomer 974P, carbomer 980 and carbomer 1342, that are commercially available under the respective Carbopol^{®} trademark names. Preferred carbomers are carbomer 974P (Carbopol^{®} 974P) and/or carbomer 980 (Carbopol^{®} 980).

Mucoadhesion of the polyacrylic acid in the ophthalmic composition depends on the pH of the composition. Therefore, the pH of the ophthalmic composition should be as defined below.

The aqueous ophthalmic composition preferably further contains panthenol, preferably dexpanthenol. The composition may contain 0.5 to 4.0 % w/v, preferably 0.8 to 3.0 % w/v, more preferably 1.0 to 3.0 % w/v panthenol, preferably dexpanthenol. The panthenol preferably contributes to the effect of the invention to improve wetting, the efficacy in the treatment of dry eyes and mucoadhesion of the ophthalmic composition.

The aqueous ophthalmic composition of the invention may further comprise one or more viscosity regulating agent. Herein, the viscosity regulating agent is other than the polyacrylic acid used in the invention. The viscosity regulating agent is a polymeric hydrophilic compound that is soluble in water, thereby increasing the viscosity of the solution. It has film-forming properties when applied to the eye, which protects the cornea and increases the remaining time of the ophthalmic composition on the cornea. The viscosity regulating agent may be a hydroxyalkylated cellulose, alkylated cellulose, chondroitin sulfate, polyvinyl alcohol, polyethylene glycol, polysaccharides, polyvinylpyrrolidone, hyaluronic acid, cross-linked hyaluronic acid, or salts of any of these. The hydroxyalkylated cellulose may be hydroxypropylcellulose or hydroxyethylcellulose. The alkylated cellulose may be methylcellulose or ethylcellulose. Further, a viscosity regulating agent may be hydroxypropylmethylcellulose (hypromellose, HPMC), however, in one embodiment, the ophthalmic composition of the invention does not contain HPMC. Two or more viscosity regulating agents may be combined in the ophthalmic composition of the invention. Among the above viscosity regulating agents, hyaluronic acid or a salt thereof and cross-linked hyaluronic acid or a salt thereof are preferred and hyaluronic acid or a salt thereof is most preferred.

The ophthalmic composition of the invention may comprise the one or more viscosity regulating agent(s), preferably said hyaluronic acid and/or a hyaluronate, in a concentration of from 0.05 to 0.3 % w/v, preferably from 0.1 to 0.2 % w/v of the ophthalmic composition. The concentration is indicated with reference to the acid form, i.e. hyaluronic acid, as opposed to salts thereof, whereby counter-cations such as sodium ions do not contribute to the centration). These concentration ranges relate to the sum of viscosity regulating agents, if two or more different are used. Within the above range, the ophthalmic composition is comfortable to apply and suppresses eye irritation. The exact concentration may be chosen so as to reach a desired viscosity of the ophthalmic composition. The viscosity regulating agent may be used alone or in combination of two or more kinds. Among the above, hyaluronic acid and salts thereof is particularly preferred from the view of film stability on the eye and suppression of eye irritation.

The molecular weight of the viscosity regulating agent may be chosen so as to adjust the viscosity of the ophthalmic composition to a desired range. The molecular weight may be within the range of from 100 000 to 10 000 000 Dalton, preferable within the range of from 500 000 to 5 000 000 Dalton, more preferably within the range of from 800 000 to 4 000 000 Dalton, and most preferably within the range of from 1 000 000 to 3 000 000 Dalton. The weight average molecular weight of the at least one viscosity regulating agent (preferably of the hyaluronic acid or hyaluronate) may be within the range of from 100 000 to 10 000 000 Dalton (100 kDa to 10 MDa), preferable within the range of from 500 000 to 5 000 000 Dalton (500 kDa to 5 MDa), more preferably within the range of from 800 000 to 4 000 000 Dalton (800 kDa to 4 MDa), and most preferably within the range of from 1 000 000 to 3 000 000 Dalton (1 MDa to 3 MDa). The one or more viscosity regulating agent preferably has such molecular weight and the content described above.

Herein, the weight average molecular weight may be determined by size exclusion chromatography (SEC) in combination with a multi angle laser light scattering (MALLS) detector (SEC-MALS).

GPC-MALLS is widely used to characterize high molecular weight (MW) polymers, which uses a differential refractometer and a multi-angle laser light scattering detector (MALLS) as detectors for gel permeation chromatogram (GPC). Separation of polymers is achieved by SEC based on different MW. The average molecular weight of an individual polymer is determined by MALLS based the differential scattering extent/angle of molecules of different MW. The GPC-MALLS method allows continuous measurement of the molecular weight and radius of gyration of each fraction separated by GPC. Principles of GPC-MALLS and protocols suited for hyaluronic acid are described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, Calif.

The aqueous ophthalmic composition of the invention may further contain suitable additives or pharmaceutical excipients. Examples of additives or excipients are stabilisers such as EDTA (ethylenediaminetetraacetic acid) or a salt thereof, tonicity agents (see further below), buffers, and preservatives. Examples of preservatives frequently used in ophthalmic compositions are quaternary ammonium compounds such as benzalkonium chloride, benzethonium chloride and polyquat, or polyhexanide. However, preferably, the ophthalmic composition of the invention does not contain a preservative, more preferably it does not contain a quaternary ammonium compound as preservative. Any preservative present may be contained at less than 0.001 % w/v of a preservative, preferably of a quaternary ammonium compound preservative, such as benzalkonium chloride.

The aqueous ophthalmic composition may contain an osmolarity agent. The term "osmolarity agent" (may also be referred to as tonicity agent) generally refers to a substance that dissolves in the water of the composition of the invention and thus contributes to the osmolarity of the aqueous composition. The term "osmolarity agent" comprises both penetrating and non-penetrating solutes, i.e. solutes that can and that cannot penetrate a membrane of an osmotic cell. Examples of osmolarity agents are inorganic soluble salts such as sodium and potassium salts (e.g. sodium chloride, potassium chloride), mono- and disaccharides (e.g. trehalose), and sugar alcohols. Examples of sugar alcohols are glycerol, mannitol, xylitol, and sorbitol. However, the content of sodium (preferably alkali metal ions in general) should be low as further described below, whereby sodium chloride, and preferably also potassium chloride (or alkali metal ions in general), should not be used as tonicity agents in the present invention. One osmolarity agent may be used alone in the ophthalmic composition of the invention, or two or more may be used in combination.

Alkali metal ions such as of sodium and potassium may disturb the gel structure of the polyacrylic acid in the composition. Therefore, such ions should be contained in a concentration sufficiently low to avoid such undesired effects. The composition of the invention should contain at most 30 mM, preferably at most 20 mM, more preferably at most 10, and even more preferably at most 5 mM alkali metal ions. In one embodiment, no alkali metal ions are present or added into the ophthalmic composition. In other embodiments, the ophthalmic composition contains at most 0.05 % w/v, preferably at most 0.01 % w/v sodium chloride. As also bivalent metal ions, such as alkaline earth metal ions, may disturb the gel structure of the polyacrylic acid in the composition, such ions should not be included into or present in the composition and/or a chelating agent for bivalent metal ions, such as EDTA, is added for chelating any such bivalent metal ions.

Accordingly, the concentration of alkali metal chloride, notably of sodium chloride and/or potassium chloride, in the composition may be 0.4 % w/v or less, preferably 0.2 % w/v or less, more preferably 0.1% w/v or less, even more preferably 0.05% w/v or less, and even more preferably 0.02% w/v or less. In other embodiments, the concentration of alkali metal chloride, notably of sodium chloride and/or potassium chloride, in the composition is 0.4 % w/v or less, preferably 0.2 % w/v or less, more preferably 0.1% w/v or less, even more preferably 0.05% w/v or less, and even more preferably 0.02% w/v or less. In further embodiments, the composition comprises alkali metal chloride, notably of sodium chloride and/or potassium chloride, in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v.

In another embodiment, the concentration of alkali metal ions, notably of sodium ions and/or potassium ions, in the composition is at most 70 mM, preferably at most 35 mM, more preferably at most 20 mM, even more preferably at most 10 mM, and most preferably at most 3 mM. In another embodiment, the concentration of alkali metal ions, notably of sodium ions and/or potassium ions, in the composition is from 2 to 35 mM, preferably from 4 to 25 mM, more preferably from 5 to 25 mM, even more preferably from 5 to 17 mM, and most preferably from 5 to 14 mM.

Alkali metals are lithium, sodium, potassium, rubidium, and cesium. However, since lithium, rubidium, and cesium are normally not contained in ophthalmic compositions, the skilled person understands that the above-indicated concentrations generally apply to sodium and potassium. If no potassium salts or ions are contained, they relate to sodium salts, such as sodium chloride.

Frequently, sodium ions may be introduced into the ophthalmic composition when setting the pH from more acidic pH values to the desired more neutral pH using NaOH solutions. Since the amount of sodium added is known in such cases from the volume and concentration of aqueous NaOH added, the content of sodium may be easily determined if other components (notably the carbomer) is used dissolved and used in its acid form.

The ophthalmic composition may generally have an osmolality of from 200 to 350 mOsm/kg, preferably 150 to 320 mOsm/kg. Herein, osmolality is determined experimentally according to Ph. Eur. 2.2.35. Preferably, the ophthalmic composition is an aqueous solution having an osmolality of from 120 to 350 mOsm/kg, preferably 150 to 300 mOsm/kg, even more preferably from 170 to 280 mOsm/kg, even more preferably from 180 to 270 mOsm/kg, and most preferably from 190 to 260 mOsm/kg.

The ophthalmic composition of the invention preferably has a dynamic viscosity of from 10 to 3000 mPa s, preferably of from 50 to 2000 mPa s, and more preferably from 100 to 1500 mPa·s. The dynamic viscosity is determined at 20°C using a rotational concentric cylinder viscometer at a shear rate of 100 s⁻¹ according to Ph. Eur. 2.2.10.

In another - preferred - embodiment, the ophthalmic composition of the invention has, at a shear rate of 100 s⁻¹, a viscosity of from 10 to 3000 mPa s, preferably of from 50 to 2000 mPa s, and more preferably from 100 to 1500 mPa·s at 25°C, determined using a rotational viscometer according to Ph. Eur. 2.2.10. In a further embodiment, the ophthalmic composition of the invention has, at a shear rate of 100 s⁻¹, a viscosity of from 10 to 400 mPa s, preferably of from 20 to 300 mPa s, and more preferably from 30 to 200 mPa·s at 25°C, determined using a rotational viscometer according to Ph. Eur. 2.2.10.

The ophthalmic composition of the invention is preferably an aqueous composition of a single phase, i.e. it is preferably not an emulsion or suspension. It preferably does not contain lipids (triacylglycerides) nor phospholipids (diacylphosphoglycerides). It should also not contain fatty acids or salts thereof.

The ophthalmic composition of the present invention may have a pH value from 5.0 to 7.5, preferably from 5.5 to 7.2, more preferably from 6.0 to 7.0. For keeping the pH stable or more stable, the ophthalmic composition may further comprise an ophthalmically acceptable buffer. Examples of an ophthalmically acceptable buffer include citrate buffer, tris (trometamol) buffer, acetate buffer, borate buffer, and phosphate buffer. Preferred buffers are citrate buffer and tris buffer. A content of the buffer in the ophthalmic composition of the invention may be from 5 to 60 mM, preferably from 10 to 50 mM, more preferably from 10 to 40 mM, and even more preferably from 10 to 30 mM.

The skilled person understands that the disclosures above on osmolality, viscosity, pH, components and their concentrations, etc. may be combined and may be jointly fulfilled by the ophthalmic composition of the invention. Thus, some preferred embodiments are as follows:
an aqueous ophthalmic composition comprising:
   (i) polyacrylic acid or a salt thereof and
   (ii) taurine,
wherein the concentration of alkali metal chloride in the composition is 0.4 % w/v or less, preferably 0.2 % w/v or less, more preferably 0.1% w/v or less, even more preferably 0.05% w/v or less, and even more preferably 0.02% w/v or less; or wherein the composition comprises alkali metal chloride in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v, and
wherein the composition is an aqueous solution has an osmolality of from 120 to 350 mOsm/kg, preferably 150 to 300 mOsm/kg, even more preferably from 170 to 280 mOsm/kg, even more preferably from 180 to 270 mOsm/kg, and most preferably from 190 to 260 mOsm/kg, and
having at a shear rate of 100 s⁻¹ a viscosity of from 10 to 400 mPa s, preferably of from 20 to 300 mPa s, and more preferably from 30 to 200 mPa·s at 25°C, determined using a rotational viscometer;
an aqueous ophthalmic composition comprising:
   (i) polyacrylic acid or a salt thereof and
   (ii) taurine,
wherein the composition comprises alkali metal chloride in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v, and
wherein the composition is an aqueous solution having an osmolality of from 150 to 300 mOsm/kg, preferably from 170 to 280 mOsm/kg, even more preferably from 180 to 270 mOsm/kg, and most preferably from 190 to 260 mOsm/kg, and
having at a shear rate of 100 s⁻¹ a viscosity of from 10 to 400 mPa s, preferably of from 20 to 300 mPa s, and more preferably from 30 to 200 mPa·s at 25°C, determined using a rotational viscometer;
an aqueous ophthalmic composition comprising:
   (i) polyacrylic acid or a salt thereof and
   (ii) taurine,
wherein the composition comprises alkali metal chloride in a concentration of from 0.02 to 0.15 % w/v, preferably from 0.03 to 0.15 % w/v, even more preferably from 0.03 to 0.1 % w/v, and most preferably from 0.03 to 0.08 % w/v, and
wherein the composition is an aqueous solution having an osmolality of from 150 to 280 mOsm/kg, preferably from 180 to 270 mOsm/kg, and
having at a shear rate of 100 s⁻¹ a viscosity of from 10 to 400 mPa s, preferably of from 20 to 200 mPa·s at 25°C, determined using a rotational viscometer;
an aqueous ophthalmic composition comprising:
   (i) polyacrylic acid or a salt thereof and
   (ii) taurine,
wherein the concentration of alkali metal ions in the composition is at most 70 mM, preferably at most 35 mM, more preferably at most 20 mM, even more preferably at most 10 mM, and most preferably at most 3 mM, or wherein the concentration of alkali metal ions in the composition is from 2 to 35 mM, preferably from 4 to 25 mM, more preferably from 5 to 25 mM, even more preferably from 5 to 17 mM, and most preferably from 5 to 14 mM, and
wherein the composition is an aqueous solution having an osmolality of from 120 to 350 mOsm/kg, preferably 150 to 300 mOsm/kg, even more preferably from 170 to 280 mOsm/kg, even more preferably from 180 to 270 mOsm/kg, and most preferably from 190 to 260 mOsm/kg, and
having at a shear rate of 100 s⁻¹ a viscosity of from 10 to 400 mPa s, preferably of from 20 to 300 mPa s, and more preferably from 30 to 200 mPa·s at 25°C, determined using a rotational viscometer;
an aqueous ophthalmic composition for use in a method of treatment or prevention of dry eyes or of epithelial defects in the cornea, said composition comprising:
   (i) polyacrylic acid or a salt thereof and
   (ii) taurine,
wherein the concentration of sodium ions in the composition is from 2 to 35 mM, preferably from 4 to 25 mM, more preferably from 5 to 25 mM, even more preferably from 5 to 17 mM, and most preferably from 5 to 14 mM, and
wherein the composition is an aqueous solution having an osmolality of from 150 to 300 mOsm/kg, preferably from 170 to 280 mOsm/kg, more preferably from 180 to 270 mOsm/kg, and most preferably from 190 to 260 mOsm/kg, and
having at a shear rate of 100 s⁻¹ a viscosity of from 20 to 300 mPa s, and preferably from 30 to 200 mPa·s at 25°C, determined using a rotational viscometer.

The skilled person understands that such embodiments may be combined with the disclosure herein on the concentrations of the polyacrylic acid or a salt thereof and with the disclosure herein on the concentrations of taurine, as well as with the disclosure of pH and other features.

The aqueous ophthalmic composition of the invention may further comprise an active pharmaceutical ingredient (API) as desired, such as antibiotic, a vitamin, an antiphlogistic agent, an antiallergic agent, an analgesic agent, and/or an agent for decreasing intra-ocular pressure or for treatment of glaucoma. Examples of an antibiotic are ofloxacin, norfloxacin, moxifloxacin and neomycin. Examples of vitamins are retinol, retinol acetate, retinol palmitate, and tocopherol acetate. Examples of analgesic agents are nepafenac, ketorolac, and diclofenac. Examples of an agent for decreasing intra-ocular pressure or for treatment of glaucoma are bimatoprost, latanoprost, travoprost, tafluprost, brinzolamide, dorzolamide, and timolol, or combinations thereof. Examples of antiallergic agents are azelastine and ketotifen.

The aqueous ophthalmic composition of the invention is for use in ophthalmology. The ophthalmic composition is in particular for use in a method of treatment or prevention of dry eyes, dry eye disease (or dry eye syndrome), keratoconjunctivitis sicca, meibomian gland dysfunction, Sjogren's syndrome, or of corneal epithelial defects. The ophthalmic composition may also be used for treating, preventing or alleviating the following symptoms which are possible symptoms of dry eye disease: foreign body sensation, blurred vision, burning sensation in eyes, burning or itching eyes, irritable eyelids, light sensitivity, redness of the whites of the eyes, painful eyes, excessive watering, visual stress due to intense vision, e.g. on computer screens, microscopes or long car journeys, symptoms caused by mechanical stress, e.g. by wearing hard or soft contact lenses or through diagnostic interventions on the eye. The dry eye symptoms may be light, moderate or severe. Use of the composition according to the invention generally leads to improvements in eye symptoms, for example to improvement in subjective comfort (more), eye redness (less), eye irritation or burning (less), tear film deficiencies (corrected), longer tear break-up time (TBUT, minimally 15 seconds), or resistance to dryness. The composition may be applied to the closed eye lid (e.g. as a spray), to the inner side of the lower eye lid, or be instilled on the cornea (drops or gel). The ophthalmic composition, preferably as eye drops or gel-like composition, may then be spread on the surface of the cornea by movement of the eye lid. The efficacy of the ophthalmic composition of the invention may be compared to conventional compositions, such as isotonic water, hyaluronate-containing eye drops, or to Visco-Vision^{®} Gel.

If further APIs are present, it may additionally be used for treating the disease for which such API is indicated. The invention also provides a method of treatment or prevention of dry eyes or of corneal epithelial defects in a subject, comprising administering said composition to an eye of the subject in need thereof.

The ophthalmic composition of the present invention is generally applied topically to a subject's eye affected by any of the indications mentioned above. The ophthalmic composition may be applied to an eye, such as the conjunctival sac of an eye, of a subject in need thereof. It may be applied by dropwise instillation into an eye, such as into the conjunctival sac of an eye. Accordingly, the ophthalmic composition may be an eye drop or gel-like formulation. The composition may be applied in a single dose. However, preferably, it is applied two or more times per day, such as three to five times per day. It may be applied to an eye, or both eyes one or more times per day over a period of from one day to several weeks (e.g. to four weeks). The ophthalmic composition of the present invention may be packaged in single-dose or multi-dose containers.

The invention also provides an aqueous composition, preferably an aqueous ophthalmic composition, comprising:
(ii) taurine such as in a concentration of from 0.2 to 3.0 % w/v or from 0.2 to 1.0 % w/v and
(iii) panthenol such as in a concentration of from 0.5 to 4.0 % w/v that may be used for the production of, or may itself be, an ophthalmic composition, e.g. for producing the aqueous ophthalmic composition of the invention. Such ophthalmic composition may or may not contain polyacrylic acid (as described above), but may otherwise be as further defined above inter alia with regard to osmolality, pH, viscosity, viscosity regulating agents, additives, and concentrations of any further component present.

The ophthalmic composition of the invention may be produced through a customary process by dissolving components (i) and (ii) and optionally any desired further component (such as component (iii) and a buffer as component (IV)) and optional additives in water, adjusting the pH with, for example hydrochloric acid or sodium hydroxide, and adding water up to the desired volume to achieve the aqueous composition with the components in the desired concentrations. Preferably, separate solutions in water of components (ii) to (iv), and optionally additives such as buffer, may be prepared and added to a solution of component (i) and any further components; then, then adjusting the pH with, for example sodium hydroxide or another base (e.g. amine), and adding water up to the desired volume. In order to easily control the content and concentration of alkali metal ions such as sodium in the composition, it is preferred to employ the polyacrylic acid and, if used, the hyaluronic acid, in their acid forms (as opposed to their alkali metal salts). The alkali metal ion concentration may then be controlled by controlling the amount of sodium hydroxide used to set the pH to the desired value. Bases other than sodium hydroxide may be used to keep the concentration of sodium at low values, such as an amine (e.g. tris base, triethanolamine, or triethylamine). The resultant solution may then be sterilized, e.g. by sterile filtration, before being packed in suitable plastic containers. Notably for compositions of gel-like texture, collapsible tubes or containers may be used as multi-dose containers.

Mucoadhesion of the aqueous ophthalmic composition may be determined according to Salzillo et al., Carbohydrate Polymers, Volume 153, 20 November 2016, 275-283. Efficacy of the aqueous ophthalmic composition on dry eyes or DED may be determined or approximated using cell culture models as described in the preclinical study of Abbate et al., Experimental Eye Research 222 (2022) 109168. The aqueous ophthalmic composition may be tested for improved (i.e. reduced) irritation to the eye using the toxicity test described by Alépée et al., Toxicology in Vitro 80 (2022) 105319.

Herein, volumes are determined and measured at room temperature.

### EXAMPLES

### Formulation Example 1: Hypotonic wetting solution

| | |
|---|---|
| Carbomer | 0.2 % w/v Carbopol^{®} 980 NF |
| Dexpanthenol | 2.0% w/v |
| Taurine | 0.5% w/v |

Sodium hyaluronate (at least. 1 MDa): 0.1%; w/v

| | |
|---|---|
| EDTA: | 0.01% w/v |
| Sorbitol | 0.25 % w/v (tonicity agent) |
| NaOH | q.s. for pH adjustment |
| Water for injection | q.s. ad 100 ml |

Target osmolality: 225-275 mOsmol/kg
Target pH: 7.0

### Example 1: Method producing the aqueous ophthalmic composition of Formulation Example 1

The manufacturing process comprises preparing two compositions (Part 1 and Part 2) which are subsequently combined.

### Part 1: carbomer solution:

% of the total amount of water for injection (WFI) (at room temperature) is transferred to a stainless steel container (adequate for heat sterilization via steam). The predetermined amount of carbopol is added to the container and dispersed by mixing. The pH is adjusted while stirring using 1M NaOH to the target pH-value. The mixture is then sterilized by autoclavation. The carbomer solution is cooled down to room temperature while mixing.

### Part 2: solution of other components:

Sodium hyaluronate, dexpanthenol, taurine, EDTA and sorbitol are added to the remaining amount of WFI and mixed until complete dissolution and the pH is adjusted to the target value. The solution is sterilized by filtration and then added to the sterilized carbomer solution. Parts 1 and 2 are then homogenized and adjusted to the final volume with WFI.

The obtained bulk pharmaceutical composition is then filled in the primary packaging containers.

### Example 2: Carbomer gel stability dependent on sodium chloride in presence and absence of taurine

### Preparations of formulations

### Formulation 1 (no taurine):

1) 750.00 g deionized water (Millipore, 18.2 MΩ) was added to a glass beaker at room temperature
2) Under magnetic stirring 2.002 g Carbopol 980NF was slowly added
3) Stirring was continued overnight (~12 hours) to obtain a clear solution
4) pH was adjusted to pH 7.0 using 1 M sodium hydroxide solution (3.66 g solution used)
5) Using deionized water (Millipore, 18.2 MΩ), the final weight was adjusted to 1000 g.
6) Final control of formulation: pH: 6.99, osmolality: 26 mOsmol/kg, appearance: slightly opaque solution

### Formulation 2 (with taurine):

1) 750.00g deionized water (Millipore, 18.2 MΩ) was added to a glass beaker at room temperature
2) Under magnetic stirring 20.08 g taurine was slowly added and the mixture was stirred for 60 minutes until completely dissolved
3) Under magnetic stirring 1.99 g Carbopol 980NF was slowly added
4) Stirring was continued overnight (~12 hours) to obtain a clear solution
5) pH was adjusted to pH 7.0 using 1 M sodium hydroxide solution (3.58 g used)
6) Using deionized water (Millipore, 18.2 MΩ) the final weight was adjusted to 1000 g.
7) Final control of formulation: pH: 6.97, osmolality: 194 mOsmol/kg, appearance: slightly opaque solution

### Experimental setup: Viscosity of gels (Formulation 1 and Formulation 2) against increasing sodium chloride concentration

1) Preparation sodium chloride stock solution 100 mg/g: 100.02 g sodium chloride was dissolved in 1000.04 g deionized water (Millipore, 18.2 MΩ)
2) 50 g of each formulation (Formulation 1 and Formulation 2) was added to a 200 mL glass beaker
3) To both beakers (Formulation 1 and Formulation 2), sodium chloride stock solution 100 mg/g was added in 12.5 mg steps.
4) After each addition of sodium chloride stock solution, the resulting viscosity and the appearance of the gel was determined.

The following table shows the amounts of NaCl added.

| **Sample** | **Weight of sample in g** | **Addition NaCl stock solution in g** | ***m*_{NaCl} / mg** | **Concentration g_{NaCl} / 100g_{Gel}** | **Percent by weight *w*_{NaCl}** | **Appearance** |
|---|---|---|---|---|---|---|
| Formulation 1 | 50.06 | 0.127 | 12.70 | **0.025** | 0.00025 | slightly opaque solution, no changes compared to pure gel observed by addition of NaCl |
| | 50.01 | 0.251 | 25.10 | **0.050** | 0.00050 | |
| | 50.03 | 0.377 | 37.71 | **0.075** | 0.00075 | |
| | 50.04 | 0.512 | 51.21 | **0.101** | 0.00101 | |
| | 50.00 | 0.744 | 74.41 | **0.147** | 0.00147 | |
| | 50.04 | 1.023 | 102.32 | **0.200** | 0.00200 | |
| | 50.01 | 1.221 | 122.12 | **0.238** | 0.00238 | |
| | 50.06 | 1.532 | 153.22 | **0.297** | 0.00297 | |
| | 50.04 | 2.014 | 201.43 | **0.387** | 0.00387 | |
| | 50.11 | 2.544 | 254.44 | **0.483** | 0.00483 | |
| Formulation 2 | 50.00 | 0.122 | 12.20 | **0.024** | 0.00024 | slightly opaque solution, no changes compared to pure gel observed by addition of |
| | 50.02 | 0.255 | 25.50 | **0.051** | 0.00051 | |
| | 50.06 | 0.381 | 38.11 | **0.076** | 0.00076 | |
| | 50.09 | 0.503 | 50.31 | **0.099** | 0.00099 | |
| | 50.03 | 0.751 | 75.11 | **0.148** | 0.00148 | |
| | 50.04 | 1.012 | 101.22 | **0.198** | 0.00198 | |
| 50.05 | 1.258 | 125.82 | **0.245** | 0.00245 | NaCl | |
| 50.05 | 1.518 | 151.82 | **0.294** | 0.00294 | | |
| 50.02 | 1.987 | 198.73 | **0.382** | 0.00382 | | |
| 50.07 | 2.493 | 249.34 | **0.474** | 0.00474 | | |

### Viscosity measurement

Rheometer Anton Paar MCR301, plate PP25 (d=0.5mm); Measuring points: 34; Measuring point duration: 30-2 s log
Shear rate profile: d(gamma)/dt = 0.1 - 100 1/s log; gradient: 11 points/dec Temperature: 25 °C

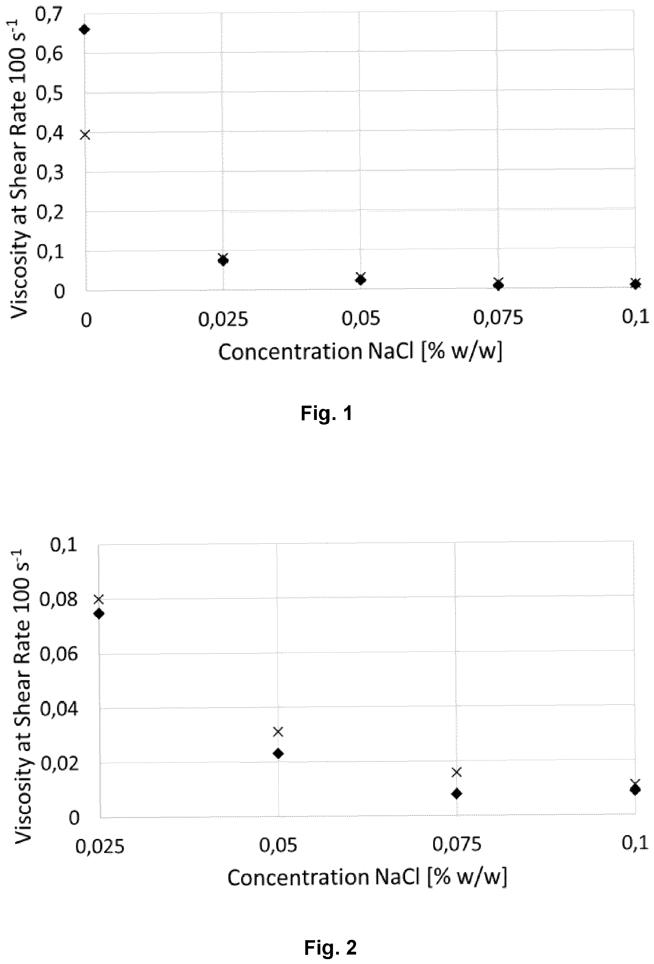

The results are shown in **Fig. 1** and **Fig. 2****.** While in the absence of NaCl a higher viscosity (more stable gel) is observed, it is vice versa in the presence of NaCl in that higher viscosities are observed (notably at elevated shear rates) in the presence of taurine. Thus, taurine can partly compensate the loss in gel stability induced by NaCl.

## Claims

1. An aqueous ophthalmic composition for use in a method of treatment or prevention of dry eyes or of epithelial defects in the cornea, said composition comprising:
(i) polyacrylic acid or a salt thereof and
(ii) taurine,
wherein the concentration of alkali metal chloride in the composition is 0.4 % w/v or less.

2. The ophthalmic composition for the use according to claim 1, further comprising (iii) hyaluronic acid or a salt thereof.

3. The ophthalmic composition for the use according to claim 1 or 2, further comprising (iv) panthenol.

4. The ophthalmic composition for the use according to claim 1 to 3, wherein the composition comprises alkali metal chloride in a concentration of 0.2 % w/v or less, preferably 0.1% w/v or less, even more preferably 0.05% w/v or less, and even more preferably 0.02% w/v or less; or wherein the composition comprises alkali metal chloride in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v.

5. An aqueous ophthalmic composition for the use according to any one of claims 1 to 4, wherein the composition is an aqueous solution having an osmolality of from 120 to 350 mOsm/kg, preferably 150 to 300 mOsm/kg, even more preferably from 170 to 280 mOsm/kg, even more preferably from 180 to 270 mOsm/kg, and most preferably from 190 to 260 mOsm/kg.

6. The ophthalmic composition for the use according to any one of claims 1 to 5, wherein the concentration of alkali metal ions in the composition is from 2 to 35 mM, preferably from 4 to 25 mM, more preferably from 5 to 25 mM, even more preferably from 5 to 17 mM, and most preferably from 5 to 14 mM.

7. The ophthalmic composition for the use according to any one of claims 1 to 6, further comprising EDTA or a salt thereof and/or further comprising a tonicity agent, such as sorbitol.

8. The ophthalmic composition for the use according to any one of claims 1 to 7, having at a shear rate of 100 s⁻¹ a viscosity of from 10 to 400 mPa s, preferably of from 20 to 300 mPa s, and more preferably from 30 to 200 mPa·s at 25 °C, determined using a rotational viscometer according to Ph. Eur. 2.2.10.

9. The ophthalmic composition for the use according to any one of claims 1 to 8, having a pH of from 5.0 to 7.5, preferably from 5.5 to 7.2, more preferably from 6.0 to 7.0.

10. The aqueous ophthalmic composition for the use according to any one of claims 1 to 9, comprising:
(i) 0.05 to 0.4 % w/v polyacrylic acid or a salt thereof,
(ii) 0.2 to 3.0 % w/v taurine, preferably 0.3 to 2.5 % w/v taurine, more preferably from 0.4 to 2.2 % w/v taurine, and
(iii) 0.05 to 0.3 % w/v hyaluronic acid or a salt thereof.

11. The aqueous ophthalmic composition for the use according to claim 10, further comprising
(iv) 0.5 to 4.0 % w/v panthenol.

12. An aqueous ophthalmic composition comprising:
(i) polyacrylic acid or a salt thereof and
(ii) taurine,
wherein the composition comprises alkali metal chloride in a concentration of from 0.01 to 0.2 % w/v, preferably from 0.02 to 0.15 % w/v, more preferably from 0.03 to 0.15 % w/v, even more preferably from 0.03 to 0.1 % w/v, and most preferably from 0.03 to 0.08 % w/v.

13. An aqueous ophthalmic composition comprising
(i) 0.1 to 0.3 % w/v polyacrylic acid or a salt thereof,
(ii) 0.2 to 3.0 % w/v taurine, preferably 0.3 to 2.5 % w/v taurine, more preferably from 0.4 to 2.2 % w/v taurine, and
(iii) optionally from 0.5 to 3.0 % w/v panthenol,
wherein the concentration of alkali metal ions in the composition is at most 70 mM, preferably at most 35 mM, more preferably at most 10 mM, and most preferably at most 4 mM; or
wherein the concentration of alkali metal ions in the composition is from 2 to 35 mM, preferably from 4 to 25 mM, more preferably from 5 to 25 mM, even more preferably from 5 to 17 mM, and most preferably from 5 to 14 mM.

14. The aqueous ophthalmic composition according to claim 12 or 13, further comprising 0.05 to 0.3 % w/v hyaluronic acid or a salt thereof.

15. The ophthalmic composition according to any one of claims 1 to 14, which is an aqueous solution of a single phase, preferably does not contain lipids (triacylglycerides) nor phospholipids (diacylphosphoglycerides), nor emulgators (such as Tween); and/or wherein the ophthalmic composition does not contain a preservative such as a quaternary ammonium compound.
